(19) **Europäisches Patentamt**
**European Patent Office**
**Office européen des brevets**

(11) **EP 4 607 521 A1**

(12) # EUROPEAN PATENT APPLICATION

(43) Date of publication:
**27.08.2025 Bulletin 2025/35**

(21) Application number: **25155454.9**

(22) Date of filing: **02.02.2025**

(51) International Patent Classification (IPC):
**G16C 20/10** $^{(2019.01)}$ **G16C 60/00** $^{(2019.01)}$

(52) Cooperative Patent Classification (CPC):
**G16C 20/10; G16C 60/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.02.2024 CN 202410203886**

(71) Applicant: **Shanghai Tosun Technology Ltd.**
**Shanghai 201804 (CN)**

(72) Inventors:
• **Mo, Mang**
  **Shanghai (CN)**
• **Xu, Jinpeng**
  **Shanghai (CN)**
• **Liu, Chu**
  **Shanghai (CN)**
• **Xie, Yueyin**
  **Shanghai (CN)**

(74) Representative: **Sun, Yanan**
**Hassler International Germany GmbH**
**Postfach 940247**
**60460 Frankfurt am Main (DE)**

(54) **METHOD AND SYSTEM FOR OBTAINING PYROLYSIS KINETICS PARAMETER OF SOLID MATERIAL AND STORAGE MEDIUM**

(57) The present disclosure relates to the technological field of pyrolysis kinetics and in particular to a method and a system for obtaining a pyrolysis kinetics parameter of a solid material and a storage medium thereof. The method of obtaining a pyrolysis kinetics parameter of a solid material includes: collecting an experimental curve of a mass loss percentage of a pyrolysate sample along with changes in temperature or time; constructing a kinetic mechanism function library and traversing each kinetic mechanism function to obtain a simulation curve of the mass loss percentage corresponding to each kinetic mechanism function along with changes in temperature or time; respectively calculating a root mean square error *(RMSE)* between the simulation curve of the mass loss percentage corresponding to each kinetic mechanism function along with changes in temperature or time and the experimental curve of the mass loss percentage along with changes in temperature or time; and sorting each *RMSE* and taking the kinetic mechanism function corresponding to a minimum *RMSE* as the pyrolysis kinetics parameter of the solid material.

EP 4 607 521 A1

## Description

### CROSS REFERENCE TO THE RELATED APPLICATIONS

This application is based upon and claims priority to Chinese Patent Application No. 202410203886.3, filed on February 23, 2024, the entire contents of which are incorporated herein by reference.

### TECHNICAL FIELD

The present disclosure relates to the technological field of pyrolysis kinetics and in particular to a method and a system for obtaining a pyrolysis kinetics parameter of a solid material and a storage medium thereof.

### BACKGROUND

Pyrolysis kinetics is a branch subject in which physical changes or chemical reaction kinetics is researched by thermal analysis or calorimetric technology. In the pyrolysis kinetics research, the most probable mechanism function is one of the important pyrolysis kinetics parameters of solid materials.

In the related arts, the most probable mechanism function is mostly obtained by subjective inference or approximate inference. In this case, the obtained most probable mechanism function is usually inaccurate and thus needs to be verified repeatedly, affecting the obtaining efficiency of the pyrolysis kinetics parameters of solid materials.

### SUMMARY

The object of the present disclosure is to provide a method and system for obtaining a pyrolysis kinetics parameter of a solid material, and a storage medium.

In order to solve the above technical problems, the present disclosure provides a method of obtaining a pyrolysis kinetics parameter of a solid material. The method includes:

collecting an experimental curve of a mass loss percentage of a pyrolysate sample along with changes in temperature or time;

constructing a kinetic mechanism function library and traversing each kinetic mechanism function to obtain a simulation curve of the mass loss percentage corresponding to each kinetic mechanism function along with changes in temperature or time;

respectively calculating a root mean square error *(RMSE)* between the simulation curve of each mass loss percentage along with changes in temperature or time and the experimental curve of the mass loss percentage along with changes in temperature or time; and,

sorting each *RMSE* and taking the kinetic mechanism function corresponding to a minimum *RMSE* as the pyrolysis kinetics parameter of the solid material.

The present disclosure has the following beneficial effects: in the method of obtaining the pyrolysis kinetics parameter of the solid material, each kinetic mechanism function is traversed sequentially based on function sequence number and in this traversal process, based on quasi-newton method, the kinetics parameters the activation energy *E* and the pre-exponential factor *A* of each kinetic mechanism function are optimized to reduce an error between the simulation curve of each mass loss percentage along with changes in temperature or time and the experimental curve of the mass loss percentage along with changes in temperature or time and thus each of the *RMSE* values is already optimized to the minimum so as to approach a theoretical minimum value, ensuring the accuracy of the sorting of the kinetic mechanism functions. Furthermore, since all kinetic mechanism functions in the function library are traversed sequentially, the global property of the most probable mechanism function is ensured and the most probable mechanism function obtained hereby has a high accuracy, thereby improving the obtaining efficiency of the pyrolysis kinetics parameter of the solid material.

Other features and advantages of the present disclosure will be described in the following description, and some of these will become apparent from the description or be understood by implementing the present disclosure. The objectives and other advantages of the present disclosure can be implemented or obtained by structures specifically indicated in the description and accompanying drawings.

To make the above purposes, features, and advantages of the present disclosure clearer and more understandable, the present disclosure is described in detail below using preferred examples with reference to the accompanying drawings.

**BRIEF DESCRIPTION OF THE DRAWINGS**

In order to more clearly describe the technical solutions in the embodiments of the present disclosure or in the prior arts, the drawings required for descriptions of the specific embodiments or the prior arts will be briefly introduced. Apparently, the drawings described hereunder are only some embodiments of the present disclosure. Those skilled in the arts can obtain other drawings based on these drawings without making creative work.

FIG. 1 is a step diagram of a method of obtaining a pyrolysis kinetics parameter of a solid material according to some embodiments of the present disclosure.
FIG. 2 is a comparison diagram of No. 3 most probable mechanism function and experimental data in cases involved in some embodiments of the present disclosure.
FIG. 3 is a comparison diagram of No. 10 non-most probable mechanism function and experimental data in cases involved in some embodiments of the present disclosure.
FIG. 4 is a principle block diagram of a system for obtaining a pyrolysis kinetics parameter of a solid material according to some embodiments of the present disclosure.
FIG. 5 is a principle block diagram of an electronic device according to some embodiments of the present disclosure.

**DETAILED DESCRIPTION OF THE EMBODIMENTS**

In order to make the object, technical solutions and advantages of the embodiments of the present disclosure clearer, the technical solutions of the present disclosure will be fully and clearly described in combination with drawings. Apparently, the embodiments described herein are only some embodiments rather than all embodiments. All other embodiments obtained by those skilled in the art based on these embodiments without making creative work shall fall within the scope of protection of the present disclosure.

In the related arts, the most probable mechanism function in the pyrolysis kinetics parameters of the solid materials is mostly obtained by subjective inference or approximate inference. In this case, the obtained most probable mechanism function is usually inaccurate and thus needs to be verified repeatedly, affecting the obtaining efficiency of the pyrolysis kinetics parameters of solid materials.

Therefore, at least one embodiment provides a method of obtaining a pyrolysis kinetics parameter of a solid material. The method includes the following steps:

collecting an experimental curve of a mass loss percentage of a pyrolysate sample along with changes in temperature or time;
constructing a kinetic mechanism function library and traversing each kinetic mechanism function to obtain a simulation curve of the mass loss percentage corresponding to each kinetic mechanism function along with changes in temperature or time;
respectively calculating a root mean square error $(RMSE)$ between the simulation curve of each mass loss percentage along with changes in temperature or time and the experimental curve of the mass loss percentage along with changes in temperature or time; and,
sorting each $RMSE$ and taking the kinetic mechanism function corresponding to a minimum $RMSE$ as the pyrolysis kinetics parameter of the solid material.

Various non-limiting implementations of the embodiments of the present disclosure will be described in details below in combination with the drawings.

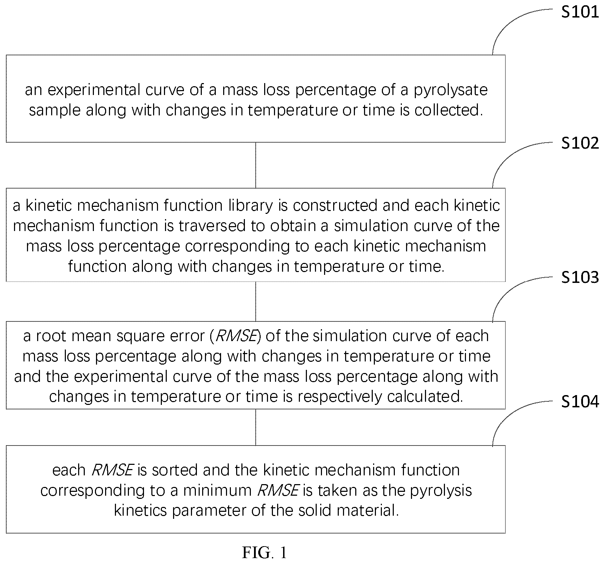

As shown in FIG. 1, some embodiments provide a method of obtaining a pyrolysis kinetics parameter of a solid material, and the method includes the following steps.

At step S101, an experimental curve of a mass loss percentage of a pyrolysate sample along with changes in temperature or time is collected.

At step S102, a kinetic mechanism function library is constructed and each kinetic mechanism function is traversed to obtain a simulation curve of the mass loss percentage corresponding to each kinetic mechanism function along with changes in temperature or time.

At step S103, a root mean square error $(RMSE)$ between the simulation curve of each mass loss percentage along with changes in temperature or time and the experimental curve of the mass loss percentage along with changes in temperature or time is respectively calculated.

At step S104, each $RMSE$ is sorted and the kinetic mechanism function corresponding to a minimum $RMSE$ is taken as the pyrolysis kinetics parameter of the solid material.

Specifically, at least one embodiment includes but not limited to a library containing 41 common kinetic mechanism functions as shown in Table 1 below, where $\alpha$ refers to a conversion rate and namely, to a conversion percentage or fraction of a reactant; and $G(\alpha)$ and $f(\alpha)$ are mechanism functions in the form of integral and differential respectively.

Table 1 Common kinetic mechanism function library

| Function sequence number | $G(\alpha)$ | $f(\alpha)$ |
|---|---|---|
| 1 | $\alpha^2$ | $\dfrac{1}{2}\alpha^{-1}$ |
| 2 | $\alpha + (1 - \alpha)\ln(1 - \alpha)$ | $[-\ln(1 - \alpha)]^{-1}$ |
| 3 | $[1 - (1 - \alpha)^{\frac{1}{2}}]^{\frac{1}{2}}$ | $4(1 - \alpha)^{\frac{1}{2}}[1 - (1 - \alpha)^{\frac{1}{2}}]^{\frac{1}{2}}$ |
| 4 | $[1 - (1 - \alpha)^{\frac{1}{2}}]^2$ | $(1 - \alpha)^{\frac{1}{2}}[1 - (1 - \alpha)^{\frac{1}{2}}]^{-1}$ |
| 5 | $[1 - (1 - \alpha)^{\frac{1}{3}}]^{\frac{1}{2}}$ | $6(1 - \alpha)^{\frac{2}{3}}[1 - (1 - \alpha)^{\frac{1}{3}}]^{\frac{1}{2}}$ |
| 6 | $[1 - (1 - \alpha)^{\frac{1}{3}}]^2$ | $\dfrac{3}{2}(1 - \alpha)^{\frac{2}{3}}[1 - (1 - \alpha)^{\frac{1}{3}}]^{-1}$ |
| 7 | $1 - \dfrac{2}{3}\alpha - (1 - \alpha)^{\frac{2}{3}}$ | $\dfrac{3}{2}[(1 - \alpha)^{-\frac{1}{3}} - 1]^{-1}$ |
| 8 | $[(1 + \alpha)^{\frac{1}{3}} - 1]^2$ | $\dfrac{3}{2}(1 + \alpha)^{\frac{2}{3}}[(1 + \alpha)^{\frac{1}{3}} - 1]^{-1}$ |
| 9 | $[(1 - \alpha)^{-\frac{1}{3}} - 1]^2$ | $\dfrac{3}{2}(1 - \alpha)^{\frac{4}{3}}[(1 - \alpha)^{-\frac{1}{3}} - 1]^{-1}$ |
| 10 | $[-\ln(1 - \alpha)]^{\frac{1}{4}}$ | $4(1 - \alpha)[-\ln(1 - \alpha)]^{\frac{3}{4}}$ |
| 11 | $[-\ln(1 - \alpha)]^{\frac{1}{3}}$ | $3(1 - \alpha)[-\ln(1 - \alpha)]^{\frac{2}{3}}$ |
| 12 | $[-\ln(1 - \alpha)]^{\frac{2}{5}}$ | $\dfrac{5}{2}(1 - \alpha)[-\ln(1 - \alpha)]^{\frac{3}{5}}$ |
| 13 | $[-\ln(1 - \alpha)]^{\frac{1}{2}}$ | $2(1 - \alpha)[-\ln(1 - \alpha)]^{\frac{1}{2}}$ |
| 14 | $[-\ln(1 - \alpha)]^{\frac{2}{3}}$ | $\dfrac{3}{2}(1 - \alpha)[-\ln(1 - \alpha)]^{\frac{1}{3}}$ |
| 15 | $[-\ln(1 - \alpha)]^{\frac{3}{4}}$ | $\dfrac{4}{3}(1 - \alpha)[-\ln(1 - \alpha)]^{\frac{1}{4}}$ |
| 16 | $-\ln(1 - \alpha)$ | $1 - \alpha$ |
| 17 | $[-\ln(1 - \alpha)]^{\frac{3}{2}}$ | $\dfrac{2}{3}(1 - \alpha)[-\ln(1 - \alpha)]^{-\frac{1}{2}}$ |
| 18 | $[-\ln(1 - \alpha)]^2$ | $\dfrac{1}{2}(1 - \alpha)[-\ln(1 - \alpha)]^{-1}$ |

(continued)

| Function sequence number | $G(\alpha)$ | $f(\alpha)$ |
|---|---|---|
| 19 | $[-\ln(1-\alpha)]^3$ | $\frac{1}{3}(1-\alpha)[-\ln(1-\alpha)]^{-2}$ |
| 20 | $[-\ln(1-\alpha)]^4$ | $\frac{1}{4}(1-\alpha)[-\ln(1-\alpha)]^{-3}$ |
| 21 | $\ln\left(\frac{\alpha}{1-\alpha}\right)$ | $\alpha(1-\alpha)$ |
| 22 | $\alpha^{\frac{1}{4}}$ | $4\alpha^{\frac{3}{4}}$ |
| 23 | $\alpha^{\frac{1}{3}}$ | $3\alpha^{\frac{2}{3}}$ |
| 24 | $\alpha^{\frac{1}{2}}$ | $2\alpha^{\frac{1}{2}}$ |
| 25 | $\alpha$ | 1 |
| 26 | $\alpha^{\frac{3}{2}}$ | $\frac{2}{3}\alpha^{-\frac{1}{2}}$ |
| 27 | $\alpha^2$ | $\frac{1}{2}\alpha^{-1}$ |
| 28 | $1-(1-\alpha)^{\frac{1}{4}}$ | $4(1-\alpha)^{\frac{3}{4}}$ |
| 29 | $1-(1-\alpha)^{\frac{1}{3}}$ | $3(1-\alpha)^{\frac{2}{3}}$ |
| 30 | $3[1-(1-\alpha)^{\frac{1}{3}}]$ | $(1-\alpha)^{\frac{2}{3}}$ |
| 31 | $1-(1-\alpha)^{\frac{1}{2}}$ | $2(1-\alpha)^{\frac{1}{2}}$ |
| 32 | $2[1-(1-\alpha)^{\frac{1}{2}}]$ | $(1-\alpha)^{\frac{1}{2}}$ |
| 33 | $1-(1-\alpha)^2$ | $\frac{1}{2}(1-\alpha)^{-1}$ |
| 34 | $1-(1-\alpha)^3$ | $\frac{1}{3}(1-\alpha)^{-2}$ |
| 35 | $1-(1-\alpha)^4$ | $\frac{1}{4}(1-\alpha)^{-3}$ |
| 36 | $(1-\alpha)^{-1}$ | $(1-\alpha)^2$ |
| 37 | $(1-\alpha)^{-1}-1$ | $(1-\alpha)^2$ |

(continued)

| Function sequence number | $G(\alpha)$ | $f(\alpha)$ |
|---|---|---|
| 38 | $(1-\alpha)^{-\frac{1}{2}}$ | $2(1-\alpha)^{\frac{3}{2}}$ |
| 39 | $\ln\alpha$ | $\alpha$ |
| 40 | $\ln\alpha^2$ | $\dfrac{1}{2}\alpha$ |
| 41 | $(1-\alpha)^{-2}$ | $\dfrac{1}{2}(1-\alpha)^3$ |

In some embodiments, a method of traversing each kinetic mechanism function to obtain the simulation curve of the mass loss percentage corresponding to each kinetic mechanism function along with changes in temperature or time includes:

setting an initial activation energy $E$ and an initial pre-exponential factor $A$;
sequentially traversing each kinetic mechanism function, and simultaneously based on quasi-newton method, optimizing the initial activation energy $E$ and the initial pre-exponential factor $A$ to obtain the optimized activation energy $E$ and pre-exponential factor $A$ corresponding to each kinetic mechanism function; wherein the optimization formula is:

$$(E_{a+1}, A_{a+1}) = (E_a, A_a) - Hessian\big(RMSE(E_a, A_a)\big)^{-1}\nabla RMSE(E_a, A_a)$$

with each kinetic mechanism function as thermogravimetric simulation model, using the optimized activation energy $E$ and pre-exponential factor $A$ as parameters of each thermogravimetric simulation model to perform simulation and outputting the simulation curve of the mass loss percentage corresponding to each kinetic mechanism function along with changes in temperature or time; wherein $a$ is an iteration step number in the optimization process and $a \geq 1$.

Specifically, the initial activation energy $E$ and the initial pre-exponential factor $A$ are each assigned an initial value by human, and for example, the initial value of $E$ is 70000 and the initial value of the $A$ is e^10, with an error of $\pm 10\%$.
In the method of obtaining the pyrolysis kinetics parameter of the solid material in this embodiment, each kinetic mechanism function is traversed sequentially based on function sequence number and in this traversal process, based on quasi-newton method, the kinetics parameters the activation energy $E$ and the pre-exponential factor $A$ are optimized, and then with each kinetic mechanism function as simulation model, the optimized activation energy E and pre-exponential factor A are used as the parameters of the simulation model to obtain the simulation curve of the mass loss percentage along with changes in temperature or time and then with the *RMSE* of the experimental curve of the mass loss percentage along with changes in temperature or time and the simulation curve of the mass loss percentage along with changes in temperature or time as target function, 41 *RMSE* values are obtained; then, the *RMSE* values are sorted and the kinetic mechanism function value corresponding to the minimum *RMSE* value is the most probable mechanism function. Since the parameters the activation energy $E$ and the pre-exponential factor $A$ of the simulation model of each kinetic mechanism function are already optimized, an error between the simulation curve of each mass loss percentage along with changes in temperature or time and the experimental curve of the mass loss percentage along with changes in temperature or time is reduced and thus each of the *RMSE* values is already optimized to the minimum so as to approach a theoretical minimum value, ensuring the accuracy of the sorting of the kinetic mechanism functions. Furthermore, since all kinetic mechanism functions in the function library are traversed sequentially, the global property of the most probable mechanism function is ensured, and the most probable mechanism function obtained hereby has a high accuracy, thereby improving the obtaining efficiency of the pyrolysis kinetics parameter of the solid material.
Finally, the most probable mechanism function, the kinetics parameter the activation energy $E$ of the most probable mechanism function, the kinetics parameter the pre-exponential factor $A$ of the most probable mechanism function and the *RMSE* with the experimental curve of the mass loss percentage along with changes in temperature or time are output as the pyrolysis kinetics parameters of the solid material. The more accurate kinetic mechanism functions and kinetics parameters can help construct a more accurate application model for the industrial processes such as pyrolysis, gasification and combustion and the like and thus the internal working conditions of the reactors can be known more accurately by use of Computational Fluid Dynamics (CFD), promoting the development of the relevant reactors.

In some embodiments, a method of collecting the experimental curve of the mass loss percentage of the pyrolysate sample along with changes in temperature or time includes:

under a non-isothermal pyrolysis mode, collecting mass loss data corresponding of the pyrolysate sample at different pyrolysis heating rates as samples; and,

at each pyrolysis heating rate, obtaining the experimental curve of the mass loss percentage along with changes in temperature or time, using pyrolysis time and temperature value as inputs and the mass loss percentage as outputs.

In some embodiments, a method of calculating the *RMSE* between the simulation curve of each mass loss percentage along with changes in temperature or time and the experimental curve of the mass loss percentage along with changes in temperature or time includes:

$$RMSE(Ei, Ai) = \sqrt{\frac{\sum_{n=1}^{N}(m_{exp}(t_n) - m_{E,A}(t_n))^2}{N}}$$

wherein,

$i$ is a sequence number of the kinetic mechanism function in the function library, $1 \leq i \leq I$, and $I$ is a total number of the kinetic mechanism functions;

$t_n$ is the $n$-th moment in a thermogravimetric experiment process;

$n$ is a moment sequence in the thermogravimetric experiment process, and $1 \leq n \leq N$;

$N$ is a total moment number in the thermogravimetric experiment process;

$m_{exp}(t_n)$ is the weight of the pyrolysate sample at the moment $t_n$ in the thermogravimetric experiment process;

$m_{E,A}(t_n)$ is the weight of the pyrolysate sample at the moment $t_n$ in a thermogravimetric simulation process, when the optimized activation energy is set to $E$ and the pre-exponential factor is set to $A$.

In some embodiments, a method of sorting each *RMSE* and taking the kinetic mechanism function corresponding to the minimum *RMSE* as the pyrolysis kinetics parameter of the solid material includes:

based on *RMSE,* sorting all kinetic mechanism functions in an ascending order and taking the first-ranked kinetic mechanism function as the most probable mechanism function; and, taking the most probable mechanism function as the pyrolysis kinetics parameter of the solid material.

In some embodiments, the method of obtaining the pyrolysis kinetics parameter of the solid material further includes:

taking the activation energy E corresponding to the most probable mechanism function, the pre-exponential factor *A* corresponding to the most probable mechanism function and the minimum *RMSE* as the pyrolysis kinetics parameter of the solid material.

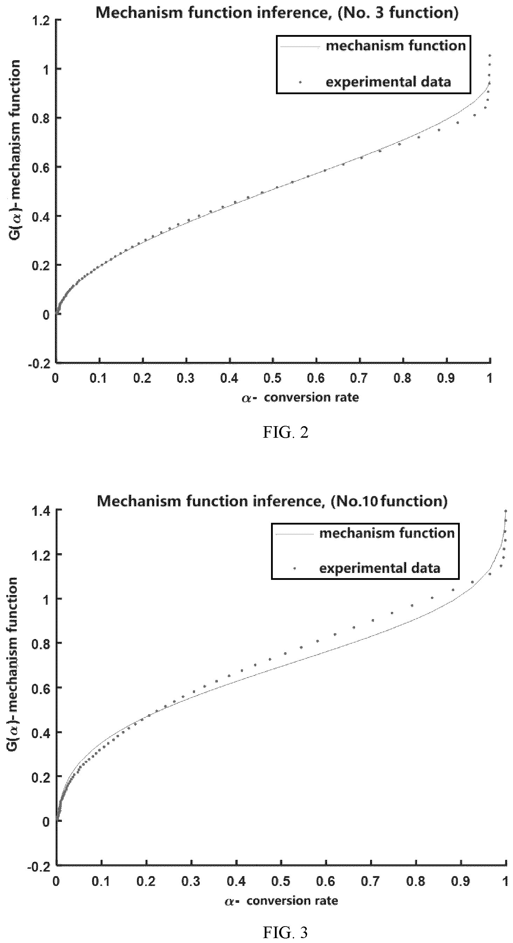

If the No. 3 mechanism function obtained by the method of obtaining the pyrolysis kinetics parameter of the solid material in this embodiment is the most probable mechanism function, the comparison with the experimental data is as shown in FIG. 2, which obviously shows that its RMSE is smaller; the comparison of No. 10 mechanism function of the non-most probable mechanism function and the experimental data is as shown in FIG. 3, which obviously shows that its RMSE is larger.

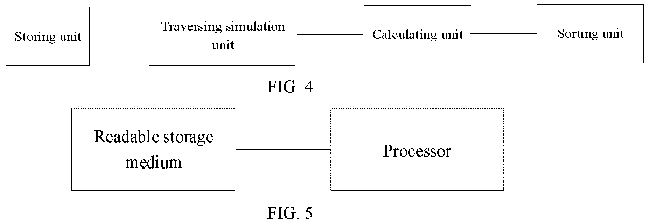

As shown in FIG. 4, some embodiments further provide a system for obtaining a pyrolysis kinetics parameter of a solid material. The system includes at least one computer device. The computer device is configured to include:

a storing unit, configured to store a collected experimental curve of a mass loss percentage of a pyrolysate sample along with changes in temperature or time;

a traversing simulation unit, configured to construct a kinetic mechanism function library and traverse each kinetic mechanism function to obtain a simulation curve of the mass loss percentage corresponding to each kinetic mechanism function along with changes in temperature or time;

a calculating unit, configured to respectively calculate a root mean square error *(RMSE)* between the simulation curve of each mass loss percentage along with changes in temperature or time and the experimental curve of the mass loss percentage along with changes in temperature or time; and,

a sorting unit, configured to sort each *RMSE* and take the kinetic mechanism function corresponding to a minimum *RMSE* as the pyrolysis kinetics parameter of the solid material.

The specific functions of the storing unit, the traversing simulation unit, the calculating unit and the sorting unit can be implemented in the computer device.

In some embodiments, the storing unit stores the experimental curve, namely, under a non-isothermal pyrolysis mode, mass loss data corresponding of the pyrolysate sample at different pyrolysis heating rates is collected as samples; and, at each pyrolysis heating rate, obtaining and storing the experimental curve of the mass loss percentage along with changes in temperature or time, using pyrolysis time and temperature value as inputs and the mass loss percentage as outputs.

In some embodiments, the storing unit is further configured to store the kinetic mechanism function library, the simulation curve of the mass loss percentage along with changes in temperature or time, the *RMSEs* and a sorting result of the *RMSEs.*

In some embodiments, the storing unit can be thought of as one data storage space.

In some embodiments, the traversing simulation unit constructs the kinetic mechanism function library and traverses each kinetic mechanism function to obtain the simulation curve of the mass loss percentage corresponding to each kinetic mechanism function along with changes in temperature or time, namely, a method of traversing each kinetic mechanism function to obtain the simulation curve of the mass loss percentage corresponding to each kinetic mechanism function along with changes in temperature or time includes: setting an initial activation energy *E* and an initial pre-exponential factor *A*; sequentially traversing each kinetic mechanism function, and simultaneously based on quasi-newton method, optimizing the initial activation energy *E* and the initial pre-exponential factor *A* to obtain the optimized activation energy *E* and pre-exponential factor *A* corresponding to each kinetic mechanism function; wherein the optimization aims to minimize the function *RMSE(E,A)* in the following optimization formula:

$$(E_{a+1}, A_{a+1}) = (E_a, A_a) - Hessian\big(RMSE(E_a, A_a)\big)^{-1} \nabla RMSE(E_a, A_a)$$

wherein,

$E_{a+1}$ represents an activation energy value of the (a+1)-th step of iteration during the optimization process, which is dependent on an activation energy value of the a-th step and gradient information;

$E_a$ represents the activation energy value of the a-th step of iteration during the optimization process, and when a=1, the initial activation energy value is taken;

$A_{a+1}$ represents a pre-exponential factor value of the (a+1)-th step of iteration during the optimization process, which is dependent on a pre-exponential factor value of the a-th step and gradient information;

$A_a$ represents the pre-exponential factor value of the a-th step of iteration during the optimization process and when a=1, the initial pre-exponential factor value is taken;

$RMSE(E_a, A_a)$ represents an *RMSE* between the simulation curve and the experimental curve of the mass loss percentage along with changes in temperature or time, when the activation energy is set to *Ea* and the pre-exponential factor is set to *Aa*; and,

with each kinetic mechanism function as thermogravimetric simulation model, using the optimized activation energy *E* and pre-exponential factor *A* as parameters of each thermogravimetric simulation model to perform simulation and outputting the simulation curve of the mass loss percentage corresponding to each kinetic mechanism function along with changes in temperature or time; wherein,

*a* is an iteration step number in the optimization process and $a \geq 1$.

In some embodiments, the calculating unit respectively calculates a root mean square error *(RMSE)* between the simulation curve of each mass loss percentage along with changes in temperature or time and the experimental curve of the mass loss percentage along with changes in temperature or time, namely,

a method of calculating the *RMSE* between the simulation curve of each mass loss percentage along with changes in temperature or time and the experimental curve of the mass loss percentage along with changes in temperature or time includes:

$$RMSE(Ei, Ai) = \sqrt{\frac{\sum_{n=1}^{N}(m_{exp}(t_n) - m_{E,A}(t_n))^2}{N}}$$

wherein,

*i* is a sequence number of the kinetic mechanism function in the function library, $1 \leq i \leq I$, and *I* is a total number of the

kinetic mechanism functions;

$t_n$ is the *n*-th moment in a thermogravimetric experiment process;

n is a moment sequence in the thermogravimetric experiment process, and $1 \leq n \leq N$;

*N* is a total moment number in the thermogravimetric experiment process;

$m_{exp}(t_n)$ is the weight of the pyrolysate sample at the moment $t_n$ in the thermogravimetric experiment process;

$m_{E,A}(t_n)$ is the weight of the pyrolysate sample at the moment $t_n$ in a thermogravimetric simulation process, when the optimized activation energy is set to *E* and pre-exponential factor is set to *A*.

In some embodiments, the sorting unit sorts each *RMSE* and takes the kinetic mechanism function corresponding to the minimum *RMSE* as the pyrolysis kinetics parameter of the solid material, namely,

based *on RMSE,* all kinetic mechanism functions are sorted in an ascending order and the first-ranked kinetic mechanism function is taken as the most probable mechanism function; and

the most probable mechanism function is taken as the pyrolysis kinetics parameter of the solid material.

The electronic device in the embodiments of the present disclosure is described below from the angle of hardware processing.

Some embodiments further provide a computer readable storage medium, storing a computer program/instruction thereon, wherein the computer program/instruction is executed by a processor to perform any one of the above methods of obtaining the pyrolysis kinetics parameter of the solid material.

As shown in FIG. 5, some embodiments further provide a computer device, including a memory, a processor and a computer program stored on the memory, wherein the computer program is executed by the processor to perform any one of the above methods of obtaining the pyrolysis kinetics parameter of the solid material.

In some embodiments, the computer device and the industrial personal computer may also be used as one of the electronic devices.

The structures shown in FIG. 5 does not constitute any limitation to the electronic device, and the electronic device may further include more or less components than shown in the drawings or combine some components or have different component deployments.

In some embodiments, the communication interface may be RS232, RS485, USB interface or TYPE interface or the like, which may be connected with an external bus adapter. The communication interface may also include wired or wireless network interface. The network interface may optionally include wired interface and/or wireless interface (such as WI-FI interface, Bluetooth interface and the like), which is usually used to establish communication connection between the server and other computer devices.

The readable storage medium or the computer readable storage medium includes at least one type of memories. The memory includes flash memory, harddisk drive, multimedia card, card type memory (e.g. SD memory or the like), magnetic memory, magnetic disk or compact disk or the like. In some embodiments, the memory may be an internal storage unit in the computer device, for example, a harddisk drive of the computer device. In some other embodiments, the memory may also be an external storage device of the computer device, for example, a plug type hard disk drive, a smart media card (SMC), a secure digital (SD) card, a flash card or the like on the computer device. Furthermore, the memory may include both the internal storage unit in the computer device and the external storage device. The memory may be used to not only store an application software installed on the computer device and various types of data, for example, the codes of the computer program and the like but also temporarily store data already output or to be output.

In some embodiments, the processor may be a central processing unit (CPU), a processor, a controller, a microcontroller, a microprocessor or another data processing chip, which is used to run the program codes in the memory or process the data, for example, execute the computer program or the like.

In some embodiments, the communication bus may also be an input/output bus, which may be a Peripheral Component Interconnect (PCI) bus, or an Enhanced Industry Standard Architecture (EISA) bus or the like. The bus may include an address bus, a data bus and a control bus and the like.

Optionally, the computer device may also include a user interface, which may include a display, and an input unit, for example, a keyboard. Optionally, the user interface may also include a standard wired interface and wireless interface. Optionally, in some embodiments, the display may be an LED display, a liquid crystal display, a touch liquid crystal display and an Organic Light-Emitting Diode (OLED) touch display and the like. The display may also be appropriately referred to as display screen or display unit for displaying information processed in the computer device as well as a visual user interface.

Some embodiments further provide a computer program product including a computer program/instruction, wherein the computer program/instruction is executed by a processor to perform any one of the above methods of obtaining the pyrolysis kinetics parameter of the solid material.

In the several embodiments provided by the present disclosure, it should be understood that the disclosed device and method can be implemented another way. The above device embodiments are merely illustrative, for example, the flowcharts or block diagrams in the drawings show possible system architectures, functions and operations of the device, method, and computer program product in the several embodiments provided by the present disclosure. Thus, each block in the flowcharts or block diagrams may represent one module, one program fragment or one part of codes. The module, the program fragment or the part of codes includes one or more executable instructions for implementing the specified logic functions. It should be noted that in some alternative embodiments, the functions indicated in the blocks may also be performed in a sequence different from that indicated in the drawings. For example, two continuous blocks can be actually performed basically in parallel, and sometimes may be performed in a reverse sequence, which is dependent on the functions involved. It is further noted that each block in the block diagrams and/or flowcharts and the combinations of the blocks in the block diagrams and/or flowcharts may be implemented by a dedicated hardware-based system for executing specified functions or actions, or by combination of dedicated hardware and computer instructions.

Furthermore, the functional modules in the embodiments of the present disclosure can be integrated into one independent part, or exist as separate modules or two or more of the modules are integrated into one independent part.

The functions, when implemented by software function modules and sold or used as independent products, can be stored in one computer readable storage medium. Based on such understanding, the essence of technical solutions of the present disclosure, or a part contributing to the prior arts or a part of the technical solutions can be embodied in the form of software product. The computer software product is stored in one storage medium which includes several instructions to enable one computer device (for example, a personal computer, a server, or a network device or the like) to perform all or part of the steps of the method of each of the embodiments of the present disclosure.

Enlightened by the ideal embodiments of the present disclosure, relevant workers can, based on the contents of the specification, make various changes and modifications within the scope of protection of the technical idea of the present disclosure. The technical scope of the present disclosure is not limited to the contents of the specification but to the technical scope claimed by the claims.

**Claims**

1.    A method of obtaining a pyrolysis kinetics parameter of a solid material, comprising:

collecting an experimental curve of a mass loss percentage of a pyrolysate sample along with changes in temperature or time;
constructing a kinetic mechanism function library and traversing each kinetic mechanism function to obtain a simulation curve of the mass loss percentage corresponding to each kinetic mechanism function along with changes in temperature or time;
respectively calculating a root mean square error *(RMSE)* between the simulation curve of the mass loss percentage corresponding to each kinetic mechanism function along with changes in temperature or time and the experimental curve of the mass loss percentage along with changes in temperature or time; and
sorting each *RMSE* and taking the kinetic mechanism function corresponding to a minimum *RMSE* as the pyrolysis kinetics parameter of the solid material.

2.    The method of claim 1, wherein,
the method of collecting the experimental curve of the mass loss percentage of the pyrolysate sample along with changes in temperature or time comprises:

under a non-isothermal pyrolysis mode, collecting mass loss data corresponding of the pyrolysate sample at different pyrolysis heating rates as samples; and
at each pyrolysis heating rate, obtaining the experimental curve of the mass loss percentage along with changes in temperature or time, using pyrolysis time and temperature value as inputs and the mass loss percentage as outputs.

3.    The method of claim 1, wherein,
the method of traversing each kinetic mechanism function to obtain the simulation curve of the mass loss percentage corresponding to each kinetic mechanism function along with changes in temperature or time comprises:

setting an initial activation energy E and an initial pre-exponential factor A;
sequentially traversing each kinetic mechanism function, and simultaneously based on a quasi-newton method, optimizing the initial activation energy *E* and the initial pre-exponential factor *A* to obtain an optimized activation energy *E* and an optimized pre-exponential factor *A* corresponding to each kinetic mechanism function; wherein the optimization aims to minimize a function *RMSE(E,A)* in the following optimization formula:

$$(E_{a+1}, A_{a+1}) = (E_a, A_a) - Hessian(RMSE(E_a, A_a))^{-1} \nabla RMSE(E_a, A_a)$$

wherein,
$E_{a+1}$ represents an activation energy value of an (a+1)-th step of an iteration during an optimization process, and the $E_{a+1}$ is dependent on an activation energy value of an a-th step and gradient information;
$E_a$ represents the activation energy value of the a-th step of the iteration during the optimization process, and when a=1, the initial activation energy value is taken;
$A_{a+1}$ represents a pre-exponential factor value of the (a+1)-th step of the iteration during the optimization process, and the $A_{a+1}$ is dependent on a pre-exponential factor value of the a-th step and the gradient information;
$A_a$ represents the pre-exponential factor value of the a-th step of the iteration during the optimization process and when a=1, the initial pre-exponential factor value is taken;
$RMSE(E_a, A_a)$ represents the *RMSE* between the simulation curve and the experimental curve of the mass loss percentage along with changes in temperature or time, when the activation energy is set to *Ea* and the pre-exponential factor is set to *Aa*; and
with each kinetic mechanism function as a thermogravimetric simulation model, using the optimized activation energy *E* and the optimized pre-exponential factor A as parameters of each thermogravimetric simulation model to perform a simulation and outputting the simulation curve of the mass loss percentage corresponding to each kinetic mechanism function along with changes in temperature or time; wherein,
*a* is an iteration step number in the optimization process and a≥1.

4. The method of claim 3, wherein,

the method of calculating the RMSE between the simulation curve of the mass loss percentage corresponding to each kinetic mechanism function along with changes in temperature or time and the experimental curve of the mass loss percentage along with changes in temperature or time comprises:

$$RMSE(Ei, Ai) = \sqrt{\frac{\sum_{n=1}^{N}(m_{exp}(t_n) - m_{E,A}(t_n))^2}{N}}$$

wherein,
*i* is a sequence number of the kinetic mechanism function in the kinetic mechanism function library, $1 \le i \le I$, and *I* is a total number of the kinetic mechanism functions;
$t_n$ is an *n*-th moment in a thermogravimetric experiment process;
*n* is a moment sequence in the thermogravimetric experiment process, and $1 \le n \le N$;
*N* is a total moment number in the thermogravimetric experiment process;
$m_{exp}(t_n)$ is the weight of the pyrolysate sample at the *n*-th moment $t_n$ in the thermogravimetric experiment process;
$m_{E,A}(t_n)$ is the weight of the pyrolysate sample at the *n*-th moment $t_n$ in a thermogravimetric simulation process, when the optimized activation energy is set to *E* and the optimized pre-exponential factor is set to *A*.

5. The method of claim 1, wherein,
the method of sorting each *RMSE* and taking the kinetic mechanism function corresponding to the minimum *RMSE* as the pyrolysis kinetics parameter of the solid material comprises:

based on the *RMSE,* sorting all kinetic mechanism functions in an ascending order and taking a first-ranked kinetic mechanism function as a most probable mechanism function; and
taking the most probable mechanism function as the pyrolysis kinetics parameter of the solid material.

6. The method of claim 1, further comprising:
taking the activation energy *E* and the pre-exponential factor *A that* correspond to the most probable mechanism function, and the minimum *RMSE* as the pyrolysis kinetics parameter of the solid material.

7. A system for obtaining a pyrolysis kinetics parameter of a solid material, comprising a computer device, wherein the computer device is configured to comprise:

a storing unit, configured to store a collected experimental curve of a mass loss percentage of a pyrolysate sample along with changes in temperature or time;

a traversing simulation unit, configured to construct a kinetic mechanism function library and traverse each kinetic mechanism function to obtain a simulation curve of the mass loss percentage corresponding to each kinetic mechanism function along with changes in temperature or time;

a calculating unit, configured to respectively calculate an RMSE between the simulation curve of the mass loss percentage corresponding to each kinetic mechanism function along with changes in temperature or time and the experimental curve of the mass loss percentage along with changes in temperature or time; and

a sorting unit, configured to sort each RMSE and take the kinetic mechanism function corresponding to a minimum RMSE as the pyrolysis kinetics parameter of the solid material.

8. The system of claim 7, wherein,

the storing unit stores the experimental curve, wherein

under a non-isothermal pyrolysis mode, mass loss data corresponding of the pyrolysate sample at different pyrolysis heating rates is collected as samples; and

at each pyrolysis heating rate, obtaining and storing the experimental curve of the mass loss percentage along with changes in temperature or time, using pyrolysis time and temperature value as inputs and the mass loss percentage as outputs.

9. The system of claim 7, wherein,

the traversing simulation unit constructs the kinetic mechanism function library and traverses each kinetic mechanism function to obtain the simulation curve of the mass loss percentage corresponding to each kinetic mechanism function along with changes in temperature or time, wherein

the method of traversing each kinetic mechanism function to obtain the simulation curve of the mass loss percentage corresponding to each kinetic mechanism function along with changes in temperature or time comprises:

setting an initial activation energy $E$ and an initial pre-exponential factor $A$;

sequentially traversing each kinetic mechanism function, and simultaneously based on a quasi-newton method, optimizing the initial activation energy $E$ and the initial pre-exponential factor $A$ to obtain an optimized activation energy $E$ and an optimized pre-exponential factor $A$ corresponding to each kinetic mechanism function; wherein the optimization aims to minimize a function $RMSE(E,A)$ in the following optimization formula:

$$(E_{a+1}, A_{a+1}) = (E_a, A_a) - Hessian(RMSE(E_a, A_a))^{-1} \nabla RMSE(E_a, A_a)$$

wherein,

$E_{a+1}$ represents an activation energy value of an (a+1)-th step of an iteration during an optimization process, and the $E_{a+1}$ is dependent on an activation energy value of an a-th step and gradient information;

$E_a$ represents the activation energy value of the a-th step of the iteration during the optimization process, and when a=1, the initial activation energy value is taken;

$A_{a+1}$ represents a pre-exponential factor value of the (a+1)-th step of the iteration during the optimization process, and the $A_{a+1}$ is dependent on a pre-exponential factor value of the a-th step and the gradient information;

$A_a$ represents the pre-exponential factor value of the a-th step of the iteration during the optimization process and when a=1, the initial pre-exponential factor value is taken;

$RMSE(E_a, A_a)$ represents the RMSE between the simulation curve and the experimental curve of the mass loss percentage along with changes in temperature or time, when the activation energy is set to $E_a$ and the pre-exponential factor is set to $A_a$; and

with each kinetic mechanism function as a thermogravimetric simulation model, using the optimized activation energy $E$ and the optimized pre-exponential factor A as parameters of each thermogravimetric simulation model to perform a simulation and outputting the simulation curve of the mass loss percentage corresponding to each kinetic mechanism function along with changes in temperature or time; wherein,

$a$ is an iteration step number in the optimization process and $a \geq 1$.

10. The system of claim 7, wherein,

the calculating unit respectively calculates the *RMSE* between the simulation curve of the mass loss percentage corresponding to each kinetic mechanism function along with changes in temperature or time and the experimental curve of the mass loss percentage along with changes in temperature or time, wherein
the method of calculating the RMSE between the simulation curve of the mass loss percentage corresponding to each kinetic mechanism function along with changes in temperature or time and the experimental curve of the mass loss percentage along with changes in temperature or time comprises:

$$RMSE(Ei, Ai) = \sqrt{\frac{\sum_{n=1}^{N}(m_{exp}(t_n) - m_{E,A}(t_n))^2}{N}}$$

wherein,
$i$ is a sequence number of the kinetic mechanism function in the kinetic mechanism function library, $1 \leq i \leq I$, and $I$ is a total number of the kinetic mechanism functions;
$t_n$ is an n-th moment in a thermogravimetric experiment process;
$n$ is a moment sequence in the thermogravimetric experiment process, and $1 \leq n \leq N$;
$N$ is a total moment number in the thermogravimetric experiment process;
$m_{exp}(t_n)$ is the weight of the pyrolysate sample at the n-th moment $t_n$ in the thermogravimetric experiment process;
$m_{E,A}(t_n)$ is the weight of the pyrolysate sample at the $n$-th moment $t_n$ in a thermogravimetric simulation process, when an optimized activation energy is set to $E$ and an optimized pre-exponential factor is set to $A$.

11. The system of claim 7, wherein,

the sorting unit sorts each *RMSE* and takes the kinetic mechanism function corresponding to the minimum *RMSE* as the pyrolysis kinetics parameter of the solid material, wherein
based on the *RMSE,* all kinetic mechanism functions are sorted in an ascending order and a first-ranked kinetic mechanism function is taken as a most probable mechanism function; and
the most probable mechanism function is taken as the pyrolysis kinetics parameter of the solid material.

12. The system of claim 7, wherein,
the storing unit is further configured to store the kinetic mechanism function library, the simulation curve of the mass loss percentage along with changes in temperature or time, the *RMSE,* and a sorting result of the *RMSE.*

13. A computer readable storage medium, storing a computer program/instruction thereon, wherein the computer program/instruction is executed by at least one processor to perform the method of obtaining the pyrolysis kinetics parameter of the solid material of claim 1.

14. A computer device, comprising a memory, a processor, and a computer program stored on the memory, wherein the processor executes the computer program to perform the method of obtaining the pyrolysis kinetics parameter of the solid material of claim 1.

15. A computer program product, comprising a computer program/instruction, wherein the computer program/instruction is executed by a processor to perform the method of obtaining the pyrolysis kinetics parameter of the solid material of claim 1.

S101

an experimental curve of a mass loss percentage of a pyrolysate sample along with changes in temperature or time is collected.

S102

a kinetic mechanism function library is constructed and each kinetic mechanism function is traversed to obtain a simulation curve of the mass loss percentage corresponding to each kinetic mechanism function along with changes in temperature or time.

S103

a root mean square error (*RMSE*) of the simulation curve of each mass loss percentage along with changes in temperature or time and the experimental curve of the mass loss percentage along with changes in temperature or time is respectively calculated.

S104

each *RMSE* is sorted and the kinetic mechanism function corresponding to a minimum *RMSE* is taken as the pyrolysis kinetics parameter of the solid material.

FIG. 1

FIG. 2

FIG. 3

| Storing unit | | Traversing simulation unit | | Calculating unit | | Sorting unit |
|---|---|---|---|---|---|---|

FIG. 4

| Readable storage medium | | Processor |
|---|---|---|

FIG. 5

Europäisches
Patentamt

European
Patent Office

Office européen
des brevets

**EUROPEAN SEARCH REPORT**

**Application Number**

EP 25 15 5454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 108 985 006 A (UNIV SCIENCE & TECHNOLOGY CHINA) 11 December 2018 (2018-12-11) | 1,2,5-8, 10-15 | INV. G16C20/10 |
| A | * the whole document * * in particular; paragraph [0033] - paragraph [0034] * * claim 1 * | 3,4,9 | ADD. G16C60/00 |
| A | REZAEI H. ET AL: "A numerical and experimental study on fast pyrolysis of single woody biomass particles", APPLIED ENERGY, ELSEVIER SCIENCE PUBLISHERS, GB, vol. 198, 26 December 2016 (2016-12-26), pages 320-331, XP085022259, ISSN: 0306-2619, DOI: 10.1016/J.APENERGY.2016.11.032 * the whole document * * in particular; page 326, paragraph 2.8. * | 1-15 | |
| A | CN 117 316 309 A (ELECTRIC POWER SCIENCE RES INST OF STATE GRID ANHUI ELECTRIC POWER CO) 29 December 2023 (2023-12-29) * the whole document * * in particular; paragraphs [0008], [0017] * | 1-15 | TECHNICAL FIELDS SEARCHED (IPC) G16C |
| A | CN 111 462 831 A (UNIV CHINA GEOSCIENCES WUHAN) 28 July 2020 (2020-07-28) * the whole document * | 1-15 | |
| A | CN 115 169 083 A (UNIV SHANDONG SCIENCE & TECH) 11 October 2022 (2022-10-11) * the whole document * | 1-15 | |

-/--

The present search report has been drawn up for all claims

| Place of search | Date of completion of the search | Examiner |
|---|---|---|
| The Hague | 19 June 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 25 15 5454

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 110 544 513 A (UNIV CHINA GEOSCIENCES WUHAN) 6 December 2019 (2019-12-06) <br> * the whole document * | 1-15 | |
| A | CN 110 728 039 A (UNIV CHINA GEOSCIENCES WUHAN) 24 January 2020 (2020-01-24) <br> * the whole document * | 1-15 | |
| A | CN 117 316 303 A (NANJING UNIVERSITY OF TECHNOLOGY) 29 December 2023 (2023-12-29) <br> * the whole document * | 1-15 | |
| A | SLOPIECKA K. ET AL: "Thermogravimetric analysis and kinetic study of poplar wood pyrolysis", APPLIED ENERGY, vol. 97, 5 January 2012 (2012-01-05), pages 491-497, XP028926800, ISSN: 0306-2619, DOI: 10.1016/J.APENERGY.2011.12.056 <br> * the whole document * | 1-15 | |
| A | KIM E. ET AL: "Evaluating effects of applying different kinetic models to pyrolysis modeling of fiberglass-reinforced polymer composites", FIRE AND MATERIALS, NEW YORK, NY, US, vol. 39, no. 2, 12 February 2014 (2014-02-12), pages 153-173, XP071613224, ISSN: 0308-0501, DOI: 10.1002/FAM.2239 <br> * the whole document * | 1-15 | |

**TECHNICAL FIELDS SEARCHED (IPC)**

The present search report has been drawn up for all claims

| Place of search | Date of completion of the search | Examiner |
|---|---|---|
| The Hague | 19 June 2025 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 5454

This annex lists the patent family members relating to the patent documents cited in  the above-mentioned European search report.
The members are as contained in the European Patent Office EDP file on
The European Patent Office is in no way liable for these particulars which are merely given for the purpose of information.

19-06-2025

| Patent document cited in search report | | Publication date | Patent family member(s) | Publication date |
|---|---|---|---|---|
| CN 108985006 | A | 11-12-2018 | NONE | |
| CN 117316309 | A | 29-12-2023 | NONE | |
| CN 111462831 | A | 28-07-2020 | NONE | |
| CN 115169083 | A | 11-10-2022 | NONE | |
| CN 110544513 | A | 06-12-2019 | NONE | |
| CN 110728039 | A | 24-01-2020 | NONE | |
| CN 117316303 | A | 29-12-2023 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410203886